# EUROPÄISCHE PATENTSCHRIFT

(11) **EP 2 834 324 B1**
(45) Veröffentlichungstag und Bekanntmachung des Hinweises auf die Patenterteilung: **06.07.2016**
(21) Anmeldenummer: 13716729.2
(22) Anmeldetag: 02.04.2013
(51) Int. Cl.: C07C 7/11, B01J 19/24, C10G 9/00, C07C 4/04, B01D 53/14, B01D 53/18, C10G 9/36

(54) **VERFAHREN ZUR TRENNUNG VON OLEFINEN BEI MILDER SPALTUNG**
METHOD FOR SEPARATING OLEFINS WITH GENTLE CLEAVAGE
PROCÉDÉ DE SÉPARATION D'OLÉFINES LORS D'UN CRAQUAGE À SÉVÉRITÉ BASSE

(30) Priorität: 05.04.2012 DE 102012006992
(43) Veröffentlichungstag der Anmeldung: 11.02.2015
(73) Patentinhaber: Linde AG, 80331 München (DE)
(72) Erfinder: PHAM DUC, Tuat, 82377 Penzberg (DE)
(86) Internationale Anmeldenummer: PCT/EP2013/000973
(87) Internationale Veröffentlichungsnummer: WO 2013/149721

(56) Entgegenhaltungen:
- EP-A1- 1 413 621
- EP-A2- 1 158 038
- WO-A1-00/44694
- WO-A1-93/12200
- DE-A1-102006 045 498
- US-A- 3 923 921

## Beschreibung

Die Erfindung betrifft ein Verfahren zur Erzeugung von Olefinen, wobei ein insbesondere flüssiger kohlenwasserstoffhaltiger Einsatz in einen Spaltofen geführt wird, wo längerkettige Kohlenwasserstoffe des kohlenwasserstoffhaltigen Einsatzes in kürzerkettige Olefine, darunter Ethylen und Propylen, gespalten werden.

Gemäß einem derartigen Verfahren wird der kohlenwasserstoffhaltige Einsatz zusammen mit Dampf in einen sogenannten Spaltofen einer entsprechenden Vorrichtung geführt und dort über Wärmestrahlung soweit erwärmt, dass sich die längerkettigen Kohlenwasserstoffe des kohlenwasserstoffhaltigen Einsatzes in die kürzerkettigen Olefine spalten. Der insbesondere flüssige kohlenwasserstoffhaltige Einsatz, der im Rahmen dieser Anmeldung zum Einsatz kommt, besteht überwiegend aus gesättigten längerkettigen Kohlenwasserstoffen. Der kohlenwasserstoffhaltige Einsatz hat dabei eine Siedetemperatur von ca. -40 °C bis 600 °C. Derartige Einsätze sind beispielsweise im Stand der Technik als Naphta, atmosphärisches Gasöl, Kerosin, hydrierte oder unhydrierte schwere/hochsiedende Kohlenwasserstoffgemische bekannt. Ebenso können im Rahmen der Erfindung flüssige C3/C4-Schnitte (verflüssigtes Petroleum Gas) als Einsatz der Spaltung dienen.

Unter einem "Spaltofen" wird im Rahmen dieser Erfindung eine Spalteinheit verstanden, in der die Spaltbedingungen festgelegt sind. Es ist möglich, dass an einem Gesamtofen eine Unterteilung in zwei oder mehr Spaltöfen vorliegt. Man spricht dann häufig von Ofenzellen. Mehrere, zu einem Gesamtofen gehörende Ofenzellen weisen in der Regel voneinander unabhängige Strahlungszonen und eine gemeinsame Konvektionszone sowie einen gemeinsamen Rauchabzug auf. In diesen Fällen kann jede Ofenzelle mit eigenen Spaltbedingungen (siehe unten) betrieben werden. Jede Ofenzelle ist somit eine Spalteinheit und wird infolgedessen hier als Spaltofen bezeichnet. Der Gesamtofen weist dann mehrere Spalteinheiten oder, anders ausgedrückt, mehrere Spaltöfen auf. Liegt nur eine Ofenzelle vor, ist diese die Spalteinheit und somit der Spaltofen. Spaltöfen können zu Gruppen zusammengefasst werden, welche beispielsweise mit dem gleichen Einsatz versorgt werden. Die Spaltbedingungen innerhalb einer Ofengruppe werden in der Regel gleich oder ähnlich eingestellt. Die Erfindung kann mit einem oder mehreren Spaltöfen verwendet werden.

Durch die Spaltung des kohlenwasserstoffhaltigen Einsatzes entsteht ein bei den Temperaturen am Austritt des oder der Spaltöfen gasförmiges Gemisch, welches die zu gewinnenden Olefine, insbesondere Ethylen und Propylen, enthält. Das bei der Spaltung entstandene gasförmige Gemisch wird als Spaltgas bezeichnet. Das den Spaltofen verlassende Spaltgas muss im Folgenden durch eine Serie von Reinigungsschritten von Verunreinigungen befreit sowie in die einzelnen Kohlenwasserstofffraktionen, insbesondere Ethylen und Propylen, getrennt werden.

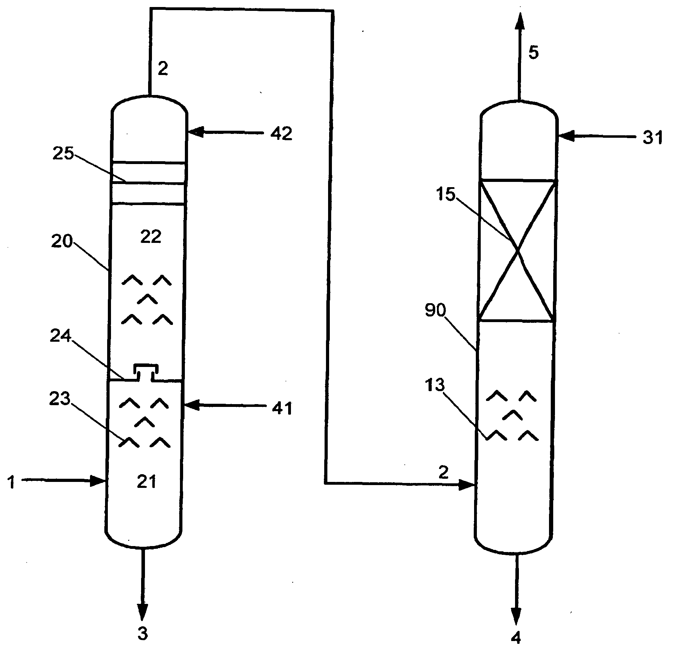

Nach dem Stand der Technik startet die Reinigung des gewonnenen Spaltgases üblicherweise wie in Figur 1 dargestellt mit einer Ölwäsche, gefolgt von einer Wasserwäsche. Weitere Reinigungsverfahren sind beispielsweise in der DE 10 2006 045 498 A1 und der EP 1 158 038 A2 offenbart, wo entsprechende Spaltgase durch ein- oder zweiteilig ausgebildete Säulen geführt werden. Ferner ist aus der WO 93/12200 A1 ein Verfahren zum Quenchen des Abstroms einer Pyrolyseeinheit und zur Entfernung schwerer Öle und Teere bekannt. Beispielsweise in der WO 100/44694 A1 ist ein Verfahren zur Vorbehandlung von Spaltgas vor der Einspeisung in einen Laugenwäscher offenbart.

Figur 1 zeigt den Beginn der Reinigungs- und Zerlegungskette eines Spaltgases, gewonnen aus einem flüssigen kohlenwasserstoffhaltigen Einsatz, nach dem Stand der Technik in einer Übersicht.

Das Spaltgas 1 wird in den unteren Abschnitt 21 einer Stoffaustauschkolonne 20 geführt, welche zwei flüssige Kohlenwasserstofffraktionen 41 und 42, die überwiegend Schweröle und Schwerbenzin enthalten, als Waschmittel verwendet. Die Stoffaustauschkolonne 20 ist in zwei Abschnitte, einen oberen Abschnitt 22 und den erwähnten unteren Abschnitt 21, getrennt. Die Trennung erfolgt mittels eines Kaminhalsbodens 24, der für die herabströmende Flüssigkeit unpassierbar und für die heraufsteigende Gasphase passierbar ist.

Die Stoffaustauschkolonne 20 weist verschiedene Elemente auf. Im unteren Abschnitt der Stoffaustauschkolonne 21 befinden sich Kaskadenböden 23. Der obere Abschnitt 22 enthält ebenfalls einige Kaskadenböden 23 sowie Elemente mit höherer Wirksamkeit wie beispielsweise Siebböden 25. Das Spaltgas 1 wird dem unteren Abschnitt der Stoffaustauschkolonne 20 aufgegeben und gerät dabei in intensiven Kontakt mit den herabströmenden Waschmitteln 41 und 42. Durch Kontakt mit den Waschmitteln 41 und 42 wird das Spaltgas vor allem abgekühlt. Schwere Ölkomponenten und feste Kokspartikel werden aus dem Spaltgas herausgewaschen. Das von den schweren Ölkomponenten und Kokspartikeln befreite Spaltgas 2 verlässt die Stoffaustauschkolonne 20 über Kopf. Vom Sumpf der Stoffaustauschkolonne 20 wird eine flüssige Phase abgezogen, die alle festen Kokspartikel sowie die schweren Ölkomponenten des Spaltgases enthält. Die Stoffaustauschkolonne 20 wird im Stand der Technik als Primary Oil Fractionater, Ölwaschkolonne oder Ölwäsche bezeichnet.

Das nunmehr von schweren Ölkomponenten und festen Kokspartikeln befreite Spaltgas 2 wird im unteren Teil einer Waschwasserkolonne 90 aufgegeben. Diese Waschwasserkolonne 90 wird auch als Wasserwäsche bezeichnet. In der Waschwasserkolonne 90 werden im unteren Teil vor allem einfache Elemente wie Kaskadenböden 13 verwendet. Im oberen Teil finden sich Elemente 15 mit höherer Wirksamkeit wie beispielsweise Siebböden, Ventilböden, Gitterpackungen, strukturierte Packungen und/oder regellose Füllkörperschüttungen.

Der Waschwasserkolonne 90 wird am Kopf kaltes Wasser 31 als Waschmittel aufgegeben. In der Waschwasserkolonne 90 wird das aufsteigende Spaltgas 2 von weiteren Verunreinigungen wie Benzinkomponenten befreit und weiter abgekühlt. Entsprechend wird vom Sumpf der Waschwasserkolonne 90 eine flüssige Fraktion 4 abgezogen, die hauptsächlich die Benzinkomponenten und Wasser enthält. Das gasförmige Produkt 5, welches die Waschwasserkolonne 90 über Kopf verlässt, ist nunmehr befreit von schweren Ölkomponenten, Benzinkomponenten sowie festen Kokspartikeln und kann im Folgenden in die einzelnen Kohlenwasserstofffraktionen, insbesondere Ethylen oder Propylen, getrennt werden (nicht weiter dargestellt).

Der vorliegenden Erfindung liegt die Aufgabe zugrunde, die Aufreinigung eines Spaltgases, wie es bei der thermischen Spaltung flüssiger kohlenwasserstoffhaltiger Einsätze entsteht, zu vereinfachen. Insbesondere soll der apparative Aufwand verringert werden.

Die gestellte Aufgabe wird durch ein Verfahren gemäß den Merkmalen des unabhängigen Patentanspruch gelöst. Weitere vorteilhafte Ausgestaltungen der Erfindung werden in den Unteransprüchen angegeben.

Die Grundidee der Erfindung ist, das bei der thermischen Spaltung des flüssigen kohlenwasserstoffhaltigen Einsatzes entstehende Spaltgas nacheinander nur mit einer benzinreichen Fraktion und einer wasserreichen Fraktion als Waschmittein aufzureinigen. Gemäß der vorliegenden Erfindung entfällt somit die komplette Ölwaschkolonne auch bei Verwendung der zuvor erläuterten kohlenwasserstoffhaltigen Einsätze wie Naphtha. Die Abtrennung der schweren Ölkomponenten, der Benzinkomponenten sowie der festen Kohlenwasserstoffpartikel erfolgt in einer einzigen Waschkolonne.

Die verwendete Waschkolonne ist ähnlich der Waschwasserkolonne bekannter Verfahren bzw. Anlagen aufgebaut und verfahrenstechnisch in einen unteren Abschnitt und einen oberen Abschnitt unterteilt. Der untere Abschnitt und der obere Abschnitt können sich durch die jeweils verwendeten Elemente unterscheiden. Das Spaltgas wird nacheinander durch den unteren Abschnitt und den oberen Abschnitt der Waschkolonne im Gegenstrom gegen die flüssigen Waschmittel, wie erwähnt eine benzinreiche Fraktion und eine wasserreiche Fraktion, geführt.

Dem oberen Abschnitt der Waschkolonne wird die wasserreiche Fraktion als Waschmittel aufgegeben. Dem unteren Abschnitt der Waschkolonne wird die benzinreiche Fraktion als Waschmittel aufgegeben. Das Spaltgas gerät im unteren Abschnitt der Waschkolonne über die dortigen Elemente in intensiven Kontakt mit der herabströmenden benzinreichen Fraktion. Die herabströmende benzinreiche Fraktion entfernt dabei aus dem Spaltgas schwere Ölkomponenten sowie feste Kokspartikel. Zusätzlich wird das aufsteigende Spaltgas gekühlt. Das aufsteigende Spaltgas gelangt anschließend in den oberen Abschnitt der Waschkolonne. In dem Spaltgas enthaltene Benzinkomponenten (aus der Spaltung und aus der als Waschmittel verwendeten benzinreichen Fraktion in dem unteren Abschnitt der Waschkolonne) werden im oberen Abschnitt mit der wasserreichen Fraktion als Waschmittel herausgewaschen. Entsprechend wird eine Benzin-Wasser-Phase erhalten Im Rahmen der vorliegenden Anmeldung wird unter einer "benzinreichen Fraktion" ein Fluid verstanden, das einen hohen Gehalt an Benzin im Sinne der unten getroffenen Definition, d.h. wenigstens 80%, insbesondere wenigstens 90%, 95% oder 99% Benzin, aufweist. Es kann sich auch um (im Wesentlichen) reines Benzin handeln. Entsprechend ist eine "wasserreiche Fraktion" eine Fraktion, die einen hohen Wassergehalt, d.h. wenigstens 80%, insbesondere wenigstens 90%, 95% oder 99% Wasser, aufweist. Eine "wasserreiche Fraktion" ist insbesondere sogenanntes Prozesswasser, also im Wesentlichen reines Wasser, das ggf. noch mit entsprechenden Hilfsstoffen beaufschlagt wird. Die "wasserreiche Fraktion" kann auch aus der erwähnten Benzin-Wasser-Phase abgetrennt werden.

Gemäß der Erfindung wird das Spaltgas direkt in die Waschkolonne als ersten Reinigungsschritt geführt. Unter einem "Reinigungsschritt" bzw. einer "Aufreinigung" wird im Rahmen der Erfindung ein Verfahrensschritt verstanden, bei dem eine oder mehrere vorgegebene Komponenten aus dem Spaltgas entfernt werden. Als "Spaltgas" wird das Gas bezeichnet, welches den Spaltofen verlässt. Unter "milden Spaltbedingungen" werden im Rahmen der Erfindung derartige Bedingungen im Spaltofen verstanden, bei denen die thermische Spaltung des kohlenwasserstoffhaltigen Einsatzes bei milder Trennschärfe, d.h. bei geringer Spaltschärfe, erfolgt.

Bei der Spaltschärfe (engl. Cracking Severity) handelt es sich um eine wichtige Kenngröße bei den hier verwendeten Verfahren zur Erzeugung von Olefinen durch Spaltung kohlenwasserstoffhaltiger Einsätze. Sie wird durch die verwendeten Spaltbedingungen bestimmt. Die Spaltbedingungen werden insbesondere beeinflusst von der Temperatur und der Verweilzeit sowie der Partialdrücke der Komponenten des kohlenwasserstoffhaltigen Einsatzes sowie des Wasserdampfs. Auch die Zusammensetzung des kohlenwasserstoffhaltigen Einsatzes und die Bauart des oder der verwendeten Spaltöfen beeinflussen die Spaltbedingungen. Aufgrund der wechselseitigen Einflüsse dieser Faktoren aufeinander ist eine Angabe der spezifischen Einzelparameter häufig nicht sinnvoll. Die Spaltbedingungen werden daher auch über das Verhältnis von Propylen zu Ethylen im Spaltgas (sogenanntes P/E-Verhältnis) angegeben.

Milde Spaltbedingungen führen beispielsweise zu einem Verhältnis von Propylen zu Ethylen von mindestens 0,7 kg/kg, insbesondere von 0,7 bis 1,6 kg/kg, bevorzugt von 0,8 bis 1,4 kg/kg, besonders bevorzugt von 0,85 bis 1,2 kg/kg am Austritt des Spaltofens. Von Vorteil können auch Spaltbedingungen sein, die zu einem Verhältnis von Propylen zu Ethylen am Austritt des Spaltofens von 0,75 bis 1,5 kg/kg oder von 0,8 bis 1,2 kg/kg oder von 0,85 bis 1,15 kg/kg führen.

Rein beispielhaft kann hierbei eine Temperatur des Spaltgases direkt am Austritt des Spaltofens zwischen 720 °C und 800 °C und sein Druck zwischen 1 barg und 4 barg, bevorzugt zwischen 2,5 barg und 4 barg, betragen. Die Druckangaben in barg beziehen sich hier auf überatmosphärischen Druck, d.h. 1 barg bedeutet 1 bar über Atmosphärendruck. Ebenso zweckmäßig ist die Zugabe von Wasserdampf zum kohlenwasserstoffhaltigen Einsatz vor dem Spaltofen in einem Massenverhältnis von Wasserdampf zu Kohlenwasserstoffen im kohlenwasserstoffhaltigen Einsatz zwischen 0,15 kg/kg und 0,45 kg/kg, bevorzugt zwischen 0,2 kg/kg und 0,35 kg/kg.

Die Erfindung kann sich insbesondere zunutze machen, dass bei der thermischen Spaltung von Kohlenwasserstoffen üblicher Zusammensetzung, wie beispielsweise Naphtha, unter milden Spaltbedingungen relativ große Mengen an (Pyrolyse-)Benzin gebildet werden. Dieses Benzin kann direkt als Waschmittel, d.h. als die "benzinreiche Fraktion" verwendet werden, so dass die Erfindung nicht auf die Bereitstellung anlagenexterner Waschmittel angewiesen ist. Das Benzin kann dabei auch in einem Anfahrbetrieb der Anlage, oder immer dann, wenn zusätzliches Benzin benötigt wird, gebildet werden. Das erfindungsgemäße Verfahren erweist sich daher als besonders kostengünstig und ermöglicht eine sinnvolle Nutzung des ohnehin anfallenden Benzins. Nicht als Waschmittel verwendetes Benzin kann an der Anlagengrenze abgegeben, fraktioniert und/oder erneut der Spaltung zugeführt werden. Die Erfindung ermöglicht damit die Erzeugung immer genau der Benzinmenge, die als Waschmittel benötigt wird. Verluste, beispielsweise durch eine nicht absolut vollständige Abtrennung aus der Benzin-Wasser-Phase (s.u.) und/oder durch Verdampfung, können jederzeit aus dem Prozess selbst ausgeglichen werden. Es kann auch vorgesehen sein, eine entsprechende Anlage nur dann mit den milden Spaltbedingungen zu betreiben, wenn Benzin als Waschmittel benötigt wird, beispielsweise bei Inbetriebnahme.

Durch die Verwendung einer einzigen Waschkolonne kann eine entsprechende Anlage platzsparender und kostengünstiger erstellt werden.

Unter "Benzin", worunter auch das erwähnte Pyrolysebenzin fällt, wird im Rahmen der Erfindung eine Kohlenwasserstofffraktion verstanden, die zu 80% bei einer Temperatur von 130 °C bei Atmosphärendruck siedet. Bei einer Temperatur oberhalb von 180 °C ist eine derartige Fraktion komplett verdampft. Entsprechend wird unter einer Benzin-Wasser-Phase ein Gemisch aus einer derartigen Kohlenwasserstofffraktion und Wasser verstanden. Die Begriffe "Benzin", "Benzinfraktion" und "Benzinphase" werden im Rahmen dieser Anmeldung synonym verwendet. Es versteht sich, dass das Benzin auch gewisse Verunreinigungen mit anderen Komponenten, beispielsweise anderen Kohlenwasserstoffen und Wasser, aufweisen kann. Die als Waschmittel verwendete benzinreiche Fraktion weist jedoch zumindest die oben erwähnten Gehalte an Benzin bzw. der den Siedepunkt von Benzin definierenden Kohlenwasserstoffe auf.

Unter einer "direkten" Zuführung des Spaltgases in die Waschkolonne wird im Rahmen der Erfindung eine direkte strömungstechnische Verbindung zwischen Spaltofen und Waschkolonne verstanden, die keine zwischengeschaltete Wäsche oder ähnliches Reinigungsschritte enthält. Gewisse bauliche Massnahme wie beispielsweise eine Rohrführung mit Knick, wo sich feste Partikel oder ähnliches sammeln können, werden im Rahmen der Erfindung nicht als zwischengeschalteter Reinigungsschritt verstanden.

Gemäß der vorliegenden Erfindung wird die Waschkolonne durch einen Boden verfahrenstechnisch in den unteren Abschnitt und den oberen Abschnitt getrennt, wobei keine Flüssigkeit den Boden von oben nach unten passieren kann. In der Erfindung wird die Waschkolonne also als Zweikreiskolonne ausgeführt. Die beiden verfahrenstechnisch getrennten Teile werden jeweils mit einem separaten Waschmittel, nämlich die erwähnten benzin- und wasserreichen Fraktionen, beaufschlagt, und verfügen über einen getrennten Flüssigkeitsabzug vom Boden der jeweiligen Teile. Dies hat den Vorteil, bei Bedarf die Waschmittel des unteren und oberen Teils unabhängig voneinander zu gestalten und zu verändern, ohne dass dabei verschiedene Kolonnen nötig sind.

Bevorzugt wird vom Boden der Waschkolonne, also dem unteren Abschnitt, eine flüssige Phase abgezogen, die die schweren Ölkomponenten und Kokspartikel enthält. Das Spaltgas verlässt in dieser Ausgestaltung die Waschkolonne gasförmig über Kopf und ist von Kokspartikeln, schweren Ölen und einer Benzinfraktion bereinigt.

Ferner wird erfindungsgemäß vom Boden, der die Waschkolonne in den unteren Abschnitt und den oberen Abschnitt trennt, eine flüssige Benzin-Wasser-Phase abgezogen. Dies erlaubt die Auftrennung dieser Phase später in eine Benzinphase und eine Wasserphase, wobei beide Phasen separat als Waschmittel in Form der benzin- bzw. wasserreichen Fraktion rückgewonnen werden.

In einer Ausgestaltung der Erfindung wird die flüssige Phase, die vom Boden der Waschkolonne, also deren unterem Abschnitt, abgezogen wird, in eine erste flüssige Phase, die die Kokspartikel enthält, und eine zweite flüssige Phase, die eine Schwerölfraktion enthält, getrennt. Dies erlaubt die getrennte Weiterverarbeitung bzw. die getrennte Rückgewinnung der einzelnen Bestandteile der flüssigen Phase aus dem Sumpf der Waschkolonne. Aus der zweiten flüssigen Phase werden zweckmäßigerweise darin eventuell vorhandene leichte Kohlenwasserstoffe (bevorzugt Kohlenwasserstoffe mit 4 bis 5 Kohlenstoffatomen) heraus gestrippt und in die Waschkolonne zurückgeführt.

Die flüssige Benzin-Wasser-Phase wird, wie erwähnt, erfindungsgemäß in eine flüssige Benzinfraktion und eine flüssige Wasserfraktion getrennt, wobei die flüssige Benzinfraktion zumindest teilweise dem unteren Teil der Waschkolonne als die benzinreiche Fraktion und die flüssige Wasserfraktion zumindest teilweise dem oberen Teil der Waschkolonne als die wasserreiche Fraktion als Waschmittel aufgegeben wird. Dies erlaubt hier ein Recycling der einzelnen Phasen.

Unter einer Schwerölfraktion wird im Rahmen der Erfindung eine Kohlenwasserstofffraktion verstanden, die zu 80% bei einer Temperatur von 460 °C bei Atmosphärendruck siedet. Bei einer Temperatur oberhalb von 560 °C ist eine derartige Fraktion komplett verdampft. Entsprechend wird unter einer Öl-Wasser-Phase ein Gemisch aus einer derartigen Schwerölfraktion und Wasser verstanden.

Im unteren Abschnitt der Waschkolonne werden bevorzugt einfache Elemente, besonders bevorzugt Kaskadenböden, und im oberen Abschnitt der Waschkolonne Elemente höherer Wirksamkeit, besonders bevorzugt Siebböden, Ventilböden, Gitterpackungen, strukturierte Packungen und/oder regellose Füllkörperschüttungen, verwendet.

Mit Hilfe der vorliegenden Erfindung gelingt es insbesondere, den apparativen Aufbau bei der Aufreinigung eines Spaltgases, wie es bei der Spaltung eines flüssigen kohlenwasserstoffhaltigen Einsatzes entsteht, zu reduzieren. Durch die vorliegende Erfindung entfällt eine gesamte Waschkolonne, der sogenannte Primary Oil Fractionator, auch für entsprechende "schwerere" Einsätze wie Naphtha. Gemäß der vorliegenden Erfindung erfolgt die Reinigung des Spaltgases von schwerem Öl, Benzinkomponenten und festen Kokspartikeln in einer Waschkolonne, bei der eine wasserreiche Fraktion als Waschmittel über Kopf in einem oberen Abschnitt aufgegeben und ferner eine benzinreiche Fraktion, die durch das Verfahren selbst erzeugt werden kann, in einem unteren Abschnitt aufgegeben wird.

Im Folgenden soll die Erfindung anhand zweier in den Figuren dargestellter Ausführungsbeispiele näher erläutert werden.

### Es zeigen

Figur 1 den Beginn der Reinigungssequenz nach dem Stand der Technik,
Figur 2 eine Ausgestaltung der Erfindung mit einfachen Schwerkraftabscheidern und
Figur 3 eine alternative Ausgestaltung der Erfindung mit einem mehrstufigen Schwerkraftabscheider

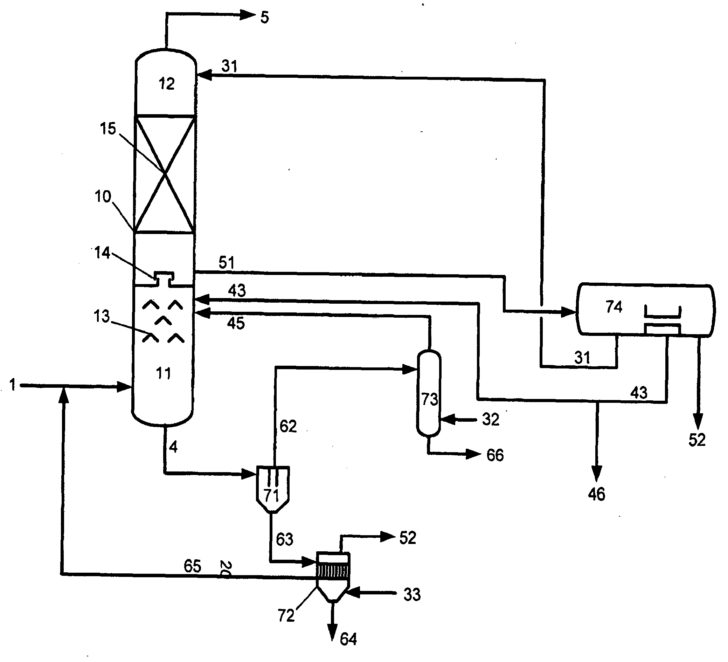

Gemäß dem Ausführungsbeispiel nach Figur 2 wird das Spaltgas 1 direkt vom Spaltofen dem unteren Abschnitt 11 der Waschkolonne 10 aufgegeben. Die Waschkolonne 10 ist durch einen Kaminhalsboden 14 verfahrenstechnisch in den unteren Abschnitt 11 und einen oberen Abschnitt 12 unterteilt. Der untere Abschnitt 11 enthält hauptsächlich einfache Elemente wie Kaskadenböden. Kaskadenböden sind in dieser Ausgestaltung Winkelelemente, die derart im unteren Teil angeordnet sind, dass die Spitze des Winkels nach oben zeigt.

Im oberen Teil 12 der Waschkolonne 10 befinden sich Elemente 15 höherer Wirksamkeit. Dies können beispielsweise strukturierte Packungen und/oder regellose Füllkörperschüttungen sein.

Dem oberen Abschnitt 12 der Waschkolonne 10 wird Wasser bzw. eine wasserreiche Fraktion 31 als Waschmittel aufgegeben. Dem unteren Abschnitt 11 der Waschkolonne 10 wird eine flüssige benzinreiche Fraktion 43 als Waschmittel aufgegeben. Das Spaltgas 1 wird in den unteren Teil 11 der Waschkolonne 10 geführt und gerät über die Elemente 13 in intensiven Kontakt mit dem herabströmenden Waschmittel 43. Die herabströmende flüssige benzinreiche Fraktion 43 entfernt dabei aus dem Spaltgas 1 schwere Öle sowie feste Kokspartikel. Zusätzlich wird das aufsteigende Spaltgas 1 gekühlt. Das aufsteigende Spaltgas 1 passiert den Kaminhalsboden 14 und gelangt in den oberen Teil 12 der Waschkolonne 10.

Der Kaminhalsboden 14 ist derart gestaltet, dass er unpassierbar für die herabströmende wasserreiche Fraktion 31 des oberen Abschnitts 12 der Waschkolonne 10 ist. Die den Kaminhalsboden 14 passierende Gasphase ist schon von allen festen Kokspartikeln sowie von schweren Ölen befreit, enthält aber noch schwere Benzinverunreinigungen. Diese Benzinverunreinigungen werden im oberen Teil mit der wasserreichen Fraktion 31 als Waschmittel herausgewaschen. Entsprechend sammelt sich auf dem Kaminhalsboden 14 eine gemischte Benzin-Wasser-Phase 51, die vom Kaminboden 14 abgezogen wird.

Die abgezogene Benzin-Wasser-Phase 51 wird einem Benzin-Wasser-Abscheider 74 zugeführt. Dort scheidet sich eine Wasserphase ab, die der Waschkolonne 10 am Kopf als wasserreiche Fraktion 31 wieder zugeführt wird. Die im Benzin-Wasser-Abscheider 74 abgeschiedene Benzinphase wird dem unteren Teil der Waschkolonne 10 als benzinreiche Fraktion 43 zugeführt. Zusätzlich wird im Benzin-Wasser-Abscheider 74 eine weitere Wasserphase 52 gewonnen, die in den Prozesswasserkreislauf der Anlage integriert wird. Zusätzlich wird ein Teil der Benzinphase (der Überschuß, der nicht für den Benzin-Kreislauf der Wasserwäsche benötigt wird) als Benzinprodukt 46 abgetrennt und zu einer weiteren Verarbeitung (beispielsweise Benzinhydrierung oder Aromatengewinnung) ausgeführt und/oder erneut gespalten.

Vom Boden der Waschkolonne 10 wird eine flüssige Phase 4 abgezogen, die sämtliche Schwerölkomponenten und Kokspartikel enthält. Die abgezogene flüssige Phase 4 wird als erstes in einen Fliehkraftabscheider (Zyklon) 71 geführt. Im Fliehkraftabscheider 71 setzen sich die festen Kokspartikel bevorzugt am Boden ab, und werden in einer flüssigen Fraktion 63 in ein Filtersystem 72 geführt. Vom Kopf des Fliehkraftabscheiders 71 wird eine flüssige Fraktion 62 abgezogen, die nur noch sehr feine feste Kokspartikel enthält. Diese wird in einen Stripper 73 geführt, wo eine gasförmige Kohlenwasserstofffraktion 45 (hauptsächlich Kohlenwasserstoffe mit 4 bis 9 Kohlenstoffatomen) durch Zufuhr von Wasserdampf 32 heraus gestrippt und der Waschkolonne 10 in den unteren Abschnitt 11 erneut aufgegeben wird. Vom Boden des Strippers 73 wird eine Schwerölphase 66 abgezogen. In dem Filtersystem 72 werden die festen Kokspartikel von der flüssigen Phase 65 getrennt, welche wieder zurück in den Spaltgasstrom 1 geführt wird. Zur Entfernung der Kokspartikel 64 aus dem Filtersystem 72 wird dieses mit Dampf 33 beaufschlagt. Dadurch ist der Strom 64, der sämtliche feste Kokspartikel enthält, frei von flüchtigen Kohlenwasserstoffen und kann somit dem Filtersystem 72 entnommen und entsorgt werden. Zusätzlich entsteht ein Abgas 52.

In dieser Ausgestaltung der Erfindung nach Figur 2 verlässt das Spaltgas 1 die Waschkolonne 10 über Kopf 5 und ist als Kopfprodukt 5 befreit von schweren ölkomponenten, Benzinkomponenten und festen Kokspartikeln. Das so gereinigte Spaltgas 5 kann dann der weiteren Tieftemperaturzerlegung zugeführt werden, wo insbesondere die Wertprodukte Ethylen und Propylen aus dem Spaltgas 5 abgetrennt werden können.

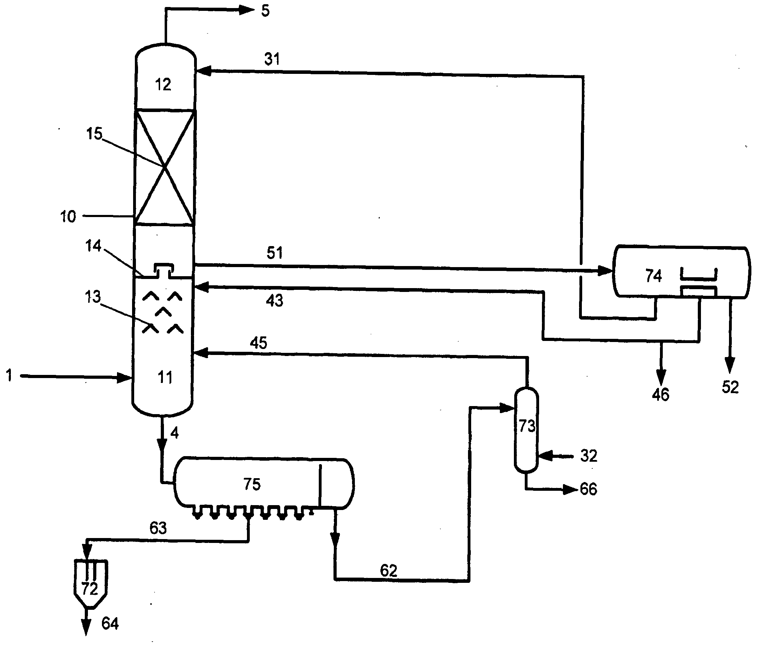

Das Ausführungsbeispiel gemäß Figur 3 ähnelt dem Ausführungsbeispiel gemäß Figur 2. Gleiche Teile und Ströme wurden hier mit gleichen Ziffern bezeichnet.

Auch hier besteht die Waschkolonne 10 aus einem unteren Abschnitt 11 und einem oberen Abschnitt 12, wobei beide Abschnitte durch einen Kaminhalsboden 14 verfahrenstechnisch getrennt sind. Dem oberen Abschnitt 12 wird, analog zu dem Ausführungsbeispiel gemäß Figur 2, Wasser 31 als Waschmittel im Kopf aufgegeben. Vom Boden des oberen Abschnitts 12 wird analog zu dem Ausführungsbeispiel aus Figur 2 eine Benzin-Wasser-Phase 51 abgezogen und ähnlich wie im Ausführungsbeispiel gemäß Figur 2 in die einzelnen Bestandteile getrennt. Dies wird hier nicht weiter dargestellt.

Im Gegensatz zum Ausführungsbeispiel nach Figur 2 wird vom Sumpf des unteren Abschnitts 11 eine Flüssigphase 4 abgezogen und direkt in einen mehrfachen Schwerkraftabscheider 75 geführt. Der mehrfache Schwerkraftabscheider 75 weist mehrere Vertiefungen auf, in denen sich die festen Kokspartikel sammeln. Aus diesen Vertiefungen wird eine flüssige Phase 63 abgezogen, die alle Kokspartikel enthält. Die flüssige Phase 63 wird in ein Filtersystem 72 geführt und analog zu dem in Figur 2 geschilderten Ausführungsbeispiel behandelt (nicht dargestellt)

Im mehrfachen Schwerkraftabscheider 75 wird ebenfalls eine Kohlenwasserstofffraktion 62 gewonnen, die nur noch sehr feine Kokspartikel enthält und analog zum Ausführungsbeispiel nach Figur 2 behandelt. Die Behandlung der Wasserfraktion 31 und der Benzin-Wasser-Phase 51 entspricht ebenfalls den Schilderungen des Ausführungsbeispieles nach Figur 2. Gleiche Ströme und Apparate wurden in beiden Ausführungsbeispielen mit gleichen Bezugszeichen versehen

## Patentansprüche

1. Verfahren zur Erzeugung von Olefinen, wobei ein kohlenwasserstoffhaltiger Einsatz in einen Spaltofen geführt wird, wo längerkettige Kohlenwasserstoffe des kohlenwasserstoffhaltigen Einsatzes zumindest teilweise in kürzerkettige Olefine, umfassend Ethylen und Propylen, gespalten werden, und wobei ein bei der Spaltung gebildetes Spaltgas (1) nacheinander durch einen unteren Abschnitt (11) und einen oberen Abschnitt (12) einer Waschkolonne (10) im Gegenstrom gegen ein flüssiges Waschmittel (43, 31) geführt wird, **dadurch gekennzeichnet,**
- **dass** in dem unteren Abschnitt (11) der Waschkolonne (10) eine benzinreiche Fraktion (43) und in dem oberen Abschnitt (12) der Waschkolonne (10) eine wasserreiche Fraktion (31) als das Waschmittel (43, 31) verwendet wird, wobei als die benzinreiche Fraktion eine Kohlenwasserstofffraktion verwendet wird, die zu 80% bei einer Temperatur von 130 °C bei Atmosphärendruck siedet,
- **dass** die Waschkolonne (10) durch einen Boden (14) verfahrenstechnisch in den unteren Abschnitt (11) und den oberen Abschnitt (12) getrennt ist, wobei der Boden (14) von oben nach unten für Flüssigkeiten unpassierbar ist,
- **dass** von dem Boden (14), der die Waschkolonne (10) in den unteren Abschnitt (11) und den oberen Abschnitt (12) trennt, eine flüssige Benzin-Wasser-Phase (51) abgezogen wird, und
- **dass** die flüssige Benzin-Wasser-Phase (51) in eine flüssige Benzinfraktion und eine flüssige Wasserfraktion getrennt wird, wobei die flüssige Benzinfraktion zumindest teilweise dem unteren Teil (11) der Waschkolonne (10) als die benzinreiche Fraktion (43) und die flüssige Wasserfraktion zumindest teilweise dem oberen Teil (12) der Waschkolonne (10) als die wasserreiche Fraktion (31) aufgegeben wird.

2. Verfahren nach Anspruch 1, **dadurch gekennzeichnet, dass** der Spaltofen derart betrieben wird, dass das Spaltgas (1) Propylen und Ethylen in einem Verhältnis zueinander von mindestens 0,7 kg/kg enthält.

3. Verfahren nach einem der Ansprüche 1 oder 2, **dadurch gekennzeichnet, dass** das Spaltgas (1) direkt am Austritt des Spaltofens eine Temperatur zwischen 720 °C und 800 °C und/oder einen Druck zwischen 1 barg und 4 barg, insbesondere zwischen 2,5 barg und 4 barg, aufweist.

4. Verfahren nach einem der vorstehenden Ansprüche, **dadurch gekennzeichnet, dass** vom Boden der Waschkolonne (10) eine flüssige Phase (4) abgezogen wird, die schwere Öle und Kokspartikel enthält, und das Spaltgas (5), welches die Waschkolonne (10) gasförmig über Kopf verlässt, von Kokspartikeln, schweren Ölen und einer Benzinfraktion bereinigt ist.

5. Verfahren nach Anspruch 4, **dadurch gekennzeichnet, dass** die flüssige Phase (4), die vom Boden der Waschkolonne (10) abgezogen wird, in eine erste flüssige Phase (64), die die Kokspartikel enthält, und eine zweite flüssige Phase (62), die eine Schwerölfraktion enthält, getrennt wird.

6. Verfahren nach Anspruch 5, **dadurch gekennzeichnet, dass** aus der zweiten flüssigen Phase (62) eventuell darin vorhandene leichte Kohlenwasserstoffe (45) heraus gestrippt und in die Waschkolonne (10) zurückgeführt werden.

7. Verfahren nach einem der vorstehenden Ansprüche, **dadurch gekennzeichnet, dass** im unteren Abschnitt (11) der Waschkolonne (10) einfache Elemente (13), insbesondere Kaskadenböden, und im oberen Abschnitt (12) der Waschkolonne (10) Elemente (15) mit höherer Wirksamkeit, insbesondere Siebböden, Ventilböden, Gitterpackungen, strukturierte Packungen und/oder Füllkörperschüttungen, verwendet werden.

8. Verfahren nach einem der vorstehenden Ansprüche, **dadurch gekennzeichnet, dass** das in dem unteren Abschnitt (11) der Waschkolonne (10) als das Waschmittel (43) verwendete benzinreiche Fraktion (43) aus dem flüssigen kohlenwasserstoffhaltigen Einsatz gewonnen wird.

## Claims

1. Process for producing olefins, in which a hydrocarbon-containing feed is fed into a cracking furnace where relatively long-chain hydrocarbons of the hydrocarbon-containing feed are at least partly cracked to form shorter-chain olefins, encompassing ethylene and propylene, and in which a cracking gas (1) formed during cracking is conveyed in succession through a lower section (11) and an upper section (12) of a scrubbing column (10) in countercurrent to a liquid scrubbing medium (43, 31), **characterized**
- **in that** a fraction (43) rich in petroleum spirit is used in the lower section (11) of the scrubbing column (10) and a water-rich fraction (31) is used in the upper section (12) of the scrubbing column (10) as the scrubbing medium (43, 31), with a hydrocarbon fraction of which 80% boils at a temperature of 130°C at atmospheric pressure being used as the fraction which is rich in petroleum spirit,
- **in that** the scrubbing column (10) is divided in process engineering terms into the lower section (11) and the upper section (12) by a tray (14), with liquids not being able to pass from the top downward through the tray (14),
- **in that** a liquid petroleum spirit-water phase (51) is taken off from the tray (14) which divides the scrubbing column (10) into the lower section (11) and the upper section (12), and
- **in that** the liquid petroleum spirit-water phase (51) is separated into a liquid petroleum spirit fraction and a liquid water fraction, with the liquid petroleum spirit fraction being at least partly introduced as the fraction (43) which is rich in petroleum spirit into the lower part (11) of the scrubbing column (10) and the liquid water fraction being at least partly introduced as the water-rich fraction (31) into the upper part (12) of the scrubbing column (10).

2. Process according to Claim 1, **characterized in that** the cracking furnace is operated in such a way that the cracking gas (1) contains propylene and ethylene in a ratio of at least 0.7 kg/kg.

3. Process according to either Claim 1 or 2, **characterized in that** the cracking gas (1) directly at the output of the cracking furnace has a temperature in the range from 720°C to 800°C and/or a pressure in the range from 1 barg to 4 barg, in particular from 2.5 barg to 4 barg.

4. Process according to any of the preceding claims, **characterized in that** a liquid phase (4) containing heavy oils and particles of carbonaceous material is taken off from the tray of the scrubbing column (10) and the cracking gas (5) which leaves the scrubbing column (10) in gaseous form at the top has been freed of particles of carbonaceous material, heavy oils and a petroleum spirit fraction.

5. Process according to Claim 4, **characterized in that** the liquid phase (4) taken off from the bottom of the scrubbing column (10) is separated into a first liquid phase (64) containing the particles of carbonaceous material and a second liquid phase (62) containing a heavy oil fraction.

6. Process according to Claim 5, **characterized in that** any light hydrocarbons (45) present in the second liquid phase (62) are stripped out of this phase and are recirculated to the scrubbing column (10).

7. Process according to any of the preceding claims, **characterized in that** simple elements (13), in particular cascade trays, are used in the lower section (11) of the scrubbing column (10) and elements (15) having a higher effectiveness, in particular sieve trays, valve trays, mesh packing, structured packing and/or beds of random packing elements, are used in the upper section (12) of the scrubbing column (10).

8. Process according to any of the preceding claims, **characterized in that** the fraction (43) which is rich in petroleum spirit which is used as the scrubbing medium (43) in the lower section (11) of the scrubbing column (10) is obtained from the liquid hydrocarbon-containing feed.

## Revendications

1. Procédé de production d'oléfines, dans lequel une charge contenant des hydrocarbures est envoyée dans un four de craquage, dans lequel des hydrocarbures à longue chaîne de la charge contenant des hydrocarbures est au moins partiellement craquée en des oléfines à chaîne plus courte, comprenant de l'éthylène et du propylène, et un gaz de craquage (1), formé lors du craquage, étant envoyé successivement dans un segment inférieur (11) et dans un segment supérieur (12) d'une colonne de lavage (10), à contre-courant par rapport à un agent de lavage liquide (43, 31), **caractérisé**
- **en ce qu'**on utilise en tant qu'agent de lavage (43, 31) dans le segment inférieur (11) de la colonne de lavage (10) une fraction (43) riche en essence et dans le segment supérieur (12) de la colonne de lavage (10) une fraction (31) riche en eau, en utilisant en tant que fraction riche en essence une fraction d'hydrocarbures, qui à raison de 80 % bout à une température de 130°C sous la pression atmosphérique,
- **en ce que** la colonne de lavage (10) est séparée par un plateau (14), conformément à la technique des procédés, en le segment inférieur (11) et le segment supérieur (12), le plateau (14) ne pouvant être traversé par des liquides de haut en bas,
- **en ce qu'**on soutire du plateau (14), qui sépare la colonne de lavage (10) en le segment inférieur (11) et le segment supérieur (12), une phase liquide essence-eau (51), et
- **en ce que** la phase liquide essence-eau (51) est séparée en une fraction essence liquide et une fraction eau liquide, la fraction essence liquide étant au moins partiellement introduite dans la partie inférieure (11) de la colonne de lavage (10) en tant que fraction (43) riche en essence, et la fraction eau liquide étant au moins partiellement introduite dans la partie supérieure (12) de la colonne de lavage (10) en tant que fraction (31) riche en eau.

2. Procédé selon la revendication 1, **caractérisé en ce que** le four de craquage est exploité de telle sorte que le gaz de craquage (1) contienne du propylène et de l'éthylène selon un rapport, l'un à l'autre, d'au moins 0,7 kg/kg.

3. Procédé selon l'une des revendications 1 ou 2, **caractérisé en ce que** le gaz de craquage (1) présente directement en sortie du four de craquage une température comprise entre 720 et 800°C et/ou une pression comprise entre 1 barg et 4 barg, en particulier entre 2,5 barg et 4 barg.

4. Procédé selon l'une des revendications précédentes, **caractérisé en ce qu'**on soutire du plateau de la colonne de lavage (10) une phase liquide (4) qui contient des huiles lourdes et des particules de coke, et le gaz de craquage (5) qui quitte la colonne de lavage (10) en tête sous forme gazeuse est purifié des particules de coke, des huiles lourdes et d'une fraction essence.

5. Procédé selon la revendication 4, **caractérisé en ce que** la phase liquide (4) qui est soutirée du plateau de la colonne de lavage (10) est séparée en une première phase liquide (64) qui contient les particules et en une deuxième phase liquide (62) qui contient une fraction huiles lourdes.

6. Procédé selon la revendication 5, **caractérisé en ce qu'**on épuise de la deuxième phase liquide (62) les hydrocarbures légers (45) qui y sont éventuellement présents, et on les renvoie dans la colonne de lavage (10).

7. Procédé selon l'une des revendications précédentes, **caractérisé en ce qu'**on utilise dans le segment inférieur (11) de la colonne de lavage (10) des éléments simples (13), en particulier des plateaux à cascades, et dans le segment supérieur (12) de la colonne de lavage (10) des éléments (15) ayant une plus grande efficacité, en particulier des plateaux perforés, des plateaux à clapets, des garnissages de grille, des garnissages structurés et/ou des masses de corps de garnissage.

8. Procédé selon l'une des revendications précédentes, **caractérisé en ce qu'**on récupère à partir de la charge liquide contenant des hydrocarbures la fraction (43) riche en essence, utilisée en tant qu'agent de lavage (43) dans le segment inférieur (11) de la colonne de lavage (10).
